Europäisches Patentamt

European Patent Office

Office européen des brevets

(11) Veröffentlichungsnummer: **0 100 000**
**A2**

## (12) EUROPÄISCHE PATENTANMELDUNG

(21) Anmeldenummer: 83106487.8

(22) Anmeldetag: 03.07.83

(51) Int. Cl.³: **F 28 G 1/12**

(30) Priorität: 24.07.82 DE 3227708

(43) Veröffentlichungstag der Anmeldung: 08.02.84
Patentblatt 84/6

(84) Benannte Vertragsstaaten: **DE FR IT**

(71) Anmelder: **Taprogge Gesellschaft mbH,
Wacholderstrasse 7, D-4000 Düsseldorf 31 (DE)**

(72) Erfinder: **Goldberg, Gerhard, Odenthaler Weg 20,
D-4000 Düsseldorf 13 (DE)**
Erfinder: **Lange, Alois, Ohlener Weg 21,
D-4030 Ratingen 1 (DE)**

(74) Vertreter: **Michelis, Theodor, Dipl.-Ing., Fliederweg 1,
D-8000 München 90 (DE)**

(54) **Schleuse zum Auffangen von Reinigungskörpern.**

(57) Schleuse (10) zum Auffangen von Reinigungskörpern in Form von Schwammgummikugeln (20), die im Kreislauf vom Kühlwasser durch die Röhren eines Wärmetauschers (2), insbesondere Kraftwerkskondensators, geführt sind, wobei die Schleuse (10) aus einem in einem zylindrischen Gehäuse (11) angeordneten Siebkörper (12) mit einer nachgeschalteten Absperrklappe (17) besteht und über eine mit einer Pumpe (14) versehenen Eintrittsleitung (13) mit dem Austrittstutzen (6) des Wärmetauschers (2) und über eine mit einem Absperrventil (18) versehene Austrittsleitung (19) mit dem Eintrittstutzen (1) des Wärmetauschers (2) in Verbindung steht. Um zum Fangen der Schwammgummikugeln (20) in der Schleuse (10) nach Ausschalten der Pumpe (14) eine Kühlwasserrückströmung und damit einen Kugelaustrag zu verhindern, ist erfindungsgemäß vorgesehen, daß innerhalb des Schleusengehäuses (11) eine die Eintrittsleitung (13) absperrbare Rückschlagklappe (26) angeordnet ist.

0100000

Taprogge Gesellschaft mbH          München, den 2.7.1983
Düsseldorf                         mein Zeichen: PT 82/71 EP

## Schleuse zum Auffangen von Reinigungskörpern

Die Erfindung betrifft eine Schleuse zum Auffangen von Reinigungskörpern in Form von Schwammgummikugeln, die im Kreislauf vom Kühlwasser durch die Röhren eines Wärmetauschers, insbesondere Dampfkraftwerkkondensators, geführt sind, wobei die Schleuse aus einem in einem zylindrischen Gehäuse angeordneten Siebkörper mit einer nachgeschalteten Absperrkappe besteht und über eine mit einer Pumpe versehenen Eintrittsleitung mit dem Austrittsstutzen des Wärmetauschers und über eine mit einem Absperrventil versehene Austrittsleitung mit dem Eintrittsstutzen des Wärmetauschers in Verbindung steht.

Danach werden also derartige Schwammgummikugeln mit dem Kühlwasser im Umlaufbetrieb durch den Wärmetauscher geführt, wobei sie hinter dem Wärmetauscher aus dem Kühlwasserstrom entfernt und vor dem Wärmetauscher dem Kühlwasser wieder zugegeben werden. Da diese Schwammgummikugeln jedoch im allgemeinen nicht kontinuierlich zur Reinigung verwendet werden, sondern nur in Intervallen, und da sie gelegentlich ausgewechselt werden müssen, ist in der Rückführleitung eine entsprechende Schleuse eingebaut, wie das z.B. aus der DE-OS 29 42 490 bekannt ist.

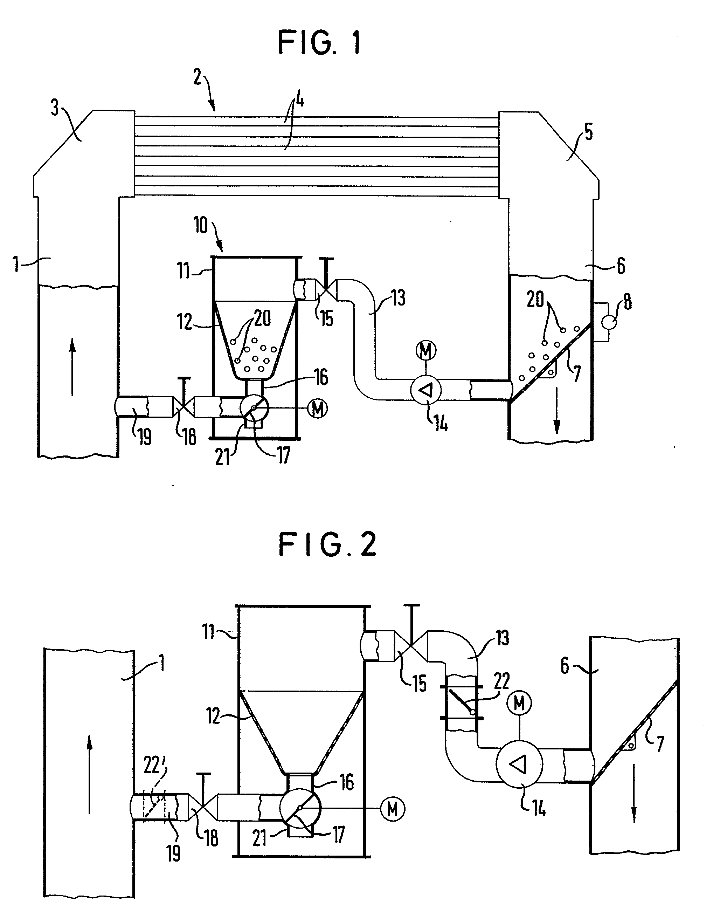

Eine bekannte Anordnung dieser Art ist schematisch in Fig. 1 dargestellt. Dabei besteht zunächst der Kühlwasserkreislauf aus dem Kühlwassereintrittstutzen 1, dem Wärmetauscher 2 mit der Eintrittskammer 3, den Wärmetauscherrohren 4, der Kühlwasseraustrittskammer 5 und dem Kühlwasseraustrittstutzen 6. Im Kühlwasseraustrittstutzen 6 ist ein schwenkbares Sieb 7 angeordnet, das in Abhängigkeit von einem Druckwächter 8 bei evtl. Verschmutzung geöffnet und damit abgespült werden kann. Parallel zum eigentlichen Wärmetauscher 2 ist nun zwischen Kühlwasseraustrittstutzen 6 und Kühlwassereintrittstutzen 1 eine Schleuse 10 angeordnet, die aus einem zylindrischen Gehäuse besteht, in das ein trichterförmiges Sieb 12 eingesetzt ist. Diese Schleuse 10 steht über eine im oberen Bereich derselben einmündende Eintrittsleitung 13 mit dem Kühlwasseraustrittstutzen 6 in Verbindung. In diese Eintrittsleitung ist eine motorgetriebene Pumpe 14 und ein motorgetriebenes Absperrventil 15 eingebaut. Das untere Ende des Siebes 12 steht über einen Rohrstutzen 16 mit einer motorgetriebenen Absperrklappe 17 in Verbindung, von der aus die mit einem Absperrventil 18 versehene Austrittsleitung 19 in den Kühlwassereintrittstutzen 1 einmündet.

Im Umlaufbetrieb der Schwammgummikugeln werden dann die Ventile 15 und 18 geöffnet, die Absperrklappe 17 in die in der Figur dargestellte Stellung gefahren und der Motor 14 eingeschaltet. Dadurch werden die vom Sieb 7 zurückgehaltenen Schwammgummikugeln 20 über die Eintrittsleitung 13 in die Schleuse 10 gefördert und - da die Absperrklappe 17 eine Verbindung zur Austrittsleitung 19 herstellt - zurück in den Eintrittstutzen 1 gefördert und dort dem Kühlwasser wieder aufgegeben.

Wenn jetzt die Schwammgummikugeln 20 in der Schleuse 10 gefangen und zurückgehalten werden sollen, muß zunächst die Absperrklappe 17 um 90$^{\circ}$ gedreht werden, so daß die direkte Verbindung zur Austrittsleitung 19 unterbrochen ist. Wenn alle Schwammgummikugeln 20 im Sieb 12 gefangen sind, kann dann die Pumpe 14 abgestellt werden. Wegen der erheblichen Druckdifferenz zwischen dem Kühlwassereintrittstutzen 1 und dem Kühlwasseraustrittstutzen 6 besteht dann aber die Gefahr, daß ein Teil des Kühlwasserstromes abgezweigt und zurück über die Austrittsleitung 19, den unteren Stutzen 21 der Absperrklappe 17, das Sieb und die Eintrittsleitung 13 zurück in den Kühlwasseraustrittstutzen 6 strömt und dabei die Schwammgummikugeln 20 wieder mit zurücknimmt. Deshalb ist es erforderlich, die Absperrventile 18 und 15 zu schließen, was mit einem erheblichen Aufwand verbunden ist, da diese Ventile meist handbetätigt sind.

Ausgehend von dieser bekannten Anordnung und Betriebsweise liegt daher der vorliegenden Erfindung die Aufgabe zugrunde, eine Schleuse zu schaffen, die einfacher und weniger störanfällig aufgebaut ist und dadurch eine sicherere Betriebsweise ermöglicht. Dies ist insbesondere dann von Vorteil, wenn die Reinigungsanlage automatisiert werden soll.

Zur Lösung dieser Aufgabe ist erfidungsgemäß vorgesehen, daß in der die Schleuse durchsetzenden Rückführleitung für die Reinigungskörper zwischen Austrittsstutzen und Eintrittsstutzen des Wärmetauschers eine Rückschlagklappe angeordnet ist.

Mit einer derartigen Anordnung einer Rückschlagklappe ist sichergestellt, daß bei Abschalten der Pumpe ein Rückstrom des Kühlwassers durch die Schleuse sicher ausgeschlossen wird, ohne daß dabei die beiden Absperrventile geschlossen werden müssen. Ein Absperren dieser Ventile ist dann nur noch erforderlich, wenn beispielsweise die Schwammgummikugeln aus der Schleuse entnommen, auf Schäden kontrolliert und ggf. durch neue Schwammgummikugeln ersetzt werden sollen.

Zweckmäßigerweise ist die Rückschlagklappe innerhalb des Schleusengehäuses zur Absperrung der Eintrittsleitung angeordnet. Sie kann dabei am innenliegenden Ende eines in die Mündung der Eintrittsleitung eingeschobenen Rohrstutzens schwnekbar befestigt sein. Es ist aber auch möglich, daß die Rückschlagklappe im Bereich der Mündung der Eintrittsleitung an der Gehäusewandung der Schleuse selbst schwenkbar befestigt ist.

Der Rohrstutzen kann dabei an einem Ende einen rechteckigen Flansch aufweisen, an dessen oberer Kante die ebenfalls rechteckige Rückschlagklappe in entsprechenden Halteösen schwenkbar aufgehängt ist.

Zur Befestigung des Rohrstutzens ist es von Vorteil, wenn der Rohrstutzen in Längsrichtung verlaufende Schlitze aufweist und dadurch in der Mündung der Eintrittsleitung elastisch federnd verklemmt ist. Es ist aber auch möglich, daß der Rohrstutzen mit der Eintrittsleitung verschraubt ist.

Die Schließbewegung der Rückschlagklappe kann dabei allein durch Schwerkraft erfolgen oder durch Federkraft unterstützt sein.

Anhand weiterer Figuren einer schematischen Zeichnung sind Aufbau und Wirkungsweise von Ausführungsbeispielen nach der Erfindung näher erläutert. Dabei zeigen:

Fig. 2     ein Ausschnitt der Gesamtanordnung mit der prinzipiellen Lage der Rückschlagklappe;

Fig. 3     die Anordnung der Rückschlagklappe innerhalb des Schleusengehäuses;

Fig. 4     die Befestigung der Rückschlagklappe direkt an der Gehäusewandung;

Fig. 5A    einen Längsschnitt im Bereich der Eintrittsleitung mit einer an einem Rohrstutzen befestigten Rückschlagklappe;

Fig. 5B    eine Aufsicht auf die an dem Rohrstutzen befestigte Rückschlagklappe und

Fig. 6     ein schematisches Bild der Gesamtanordnung
           in Fangstellung der Schleuse.

Ausgehend von der bekannten Anordnung nach Fig. 1 ist
nunmehr nach Fig. 2 erfindungsgemäß eine Rückschlagklappe 22 innerhalb der Eintrittsleitung 13 bzw. der Austrittsleitung 19, die gemeinsam die Rückführleitung für die
Reinigungskörper bilden, angeordnet. Dabei kann die
Rückschlagklappe 22 beispielsweise zwischen der Pumpe 14
und dem Absperrventil 15 in die Eintrittsleitung 13 eingebaut sein. Es ist aber auch möglich, eine entsprechende Rückschlagklappe 22' in die Austrittsleitung 19 einzubauen, wie das gestrichelt dargestellt ist.

Grundsätzlich ist dabei ein Einbau der Rückschlagklappe
an jeder beliebigen Stelle zwischen dem Austrittsstutzen 6 des Wärmetauschers 2 und dessen Eintrittsstutzen 1
möglich, wodurch erreicht werden kann, daß bei abgestellter Pumpe 14 eine Rückströmung des Kühlwassers und
damit der Reinigungskörper 20 sicher verhindert wird,
auch ohne daß die Absperrventile 15 und 18 jedesmal
geschlossen werden müssen.

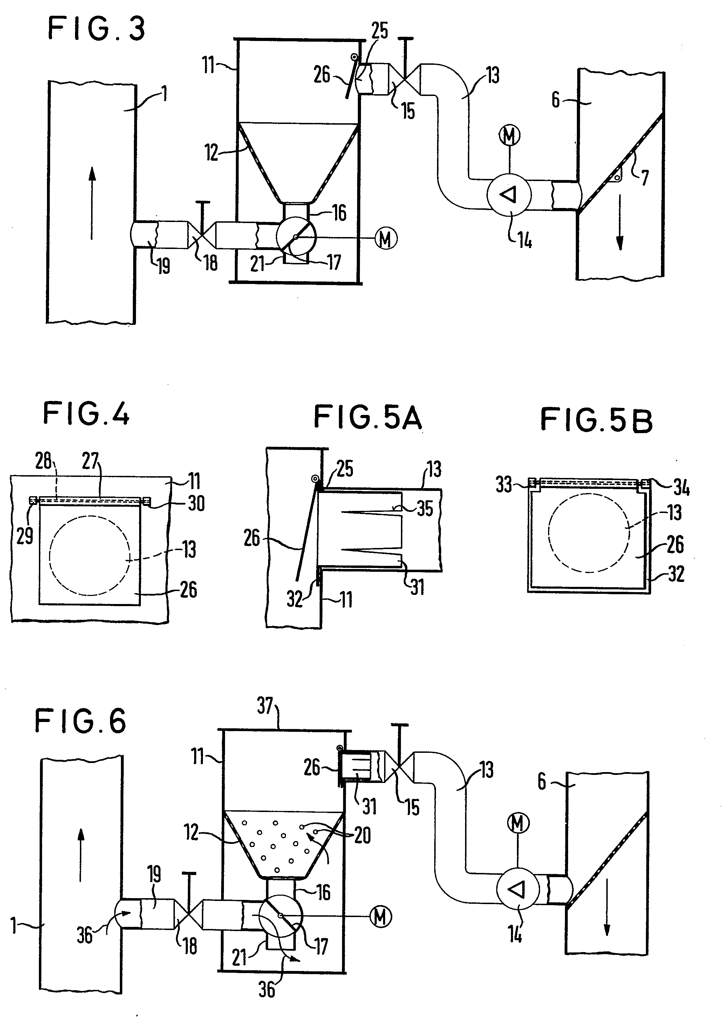

Eine besonders günstige Anordnung der Rückschlagklappe
- insbesondere für den nachträglichen Einbau - ist in
Fig. 3 dargestellt. Danach ist die Rückschlagklappe 26
innerhalb des Schleusengehäuses 11 an der Mündung 25 der
Eintrittsleitung 13 angeordnet. Diese Rückschlagklappe
26 kann - wie das in der Aufsicht nach Fig. 4 dargestellt ist - direkt an der Gehäuseinnenwandung 11 befestigt sein, indem eine einen Falz 27 an der oberen Kante der rechteckigen Rückschlagklappe 26 durchdringende Achse 28 in zwei seitlichen an der Gehäusewandung 11 befestigten Scharnieren 29 und 30 schwenkbar gehaltert ist. Die Rückschlagklappe 26 kann dabei allein durch ihre Schwerkraft schließen oder aber durch eine nicht näher dargestellte Feder in ihrer Schließwirkung unterstützt werden. Die Rückschlagklappe 26 muß dabei lediglich so dicht schließen, daß Schwammgummikugeln 20 nicht aus dem Siebkörper 12 zurück in die Eintrittsleitung 13 ausgetragen werden können. Ggf. kann die Rückschlagklappe 26 dabei leicht gewölbt entsprechend dem Radius der zylindrischen Gehäusewandung 11 ausgeführt sein.

Eine weitere zweckmäßige Befestigungsart der Rückschlagklappe 26 ist in den Fig. 5 A und B dargestellt. Danach ist zunächst eine Rohrstutzen 31 mit einem sich nach außen erstreckenden Flansch 32 am innenliegenden Ende so in die Mündung 25 der Eintrittsleitung 13 eingeschoben, daß der Flansch 32 dicht an der Gehäusewandung 11 der Schleuse 10 anliegt. Am innenliegenden Flansch 32, der zweckmäßigerweise eine rechteckige Außenkontur von etwa der Größe der Rückschlagklappe 26 aufweist, ist dann an der Oberkante in entsprechenden Scharnieren 33 und 34 die Rückschlagklappe 26 schwenkbar aufgehängt.

Zur verbesserten Halterung kann dabei der Rohrstutzen 31 in Längsrichtung verlaufende und nach der Eintrittsleitung 13 offene V-förmige Schlitze 35 aufweisen, wobei dann bei gleichem Außendurchmesser des Rohrstutzens 31 wie dem Innendurchmesser der Eintrittsleitung 13 der Rohrstutzen 31 beim Einschieben in die Leitung 13 elastisch federnd verklemmt ist. Auf diese Weise ergibt sich eine sehr einfache Montage, die jedoch eine ausreichende Halterung sicherstellt.

Selbstverständlich ist auch eine andere Befestigung dieses Rohrstutzens 31 beispielsweise durch Verschrauben - was jedoch nicht näher dargestellt ist - möglich.

In Fig. 6 ist nunmehr die Funktionsweise der Rückschlagklappe 26 in einer wesentlichen Betriebsphase gezeigt. Zum Fangen der Schwammgummikugeln 20 im Sieb 12 wird zunächst die Absperrklappe 17 um 90$^{\circ}$ in die in der Figur gezeigte Stellung gefahren, so daß der direkte Durchtritt vom Sieb 12 zur Austrittsleitung 19 für die Schwammgummikugeln unterbrochen ist. Anschließend wird die Pumpe 14 abgeschaltet. Der sich durch die Druckdifferenz zwischen dem Kühlwassereintrittstutzen 1 und dem Kühlwasseraustrittstutzen 6 evtl. aufbauende Bypass-Strömung wird nunmehr entsprechend den Pfeilen 36 über die Austrittsleitung 19, den unteren Stutzen 21 der Absperrklappe 17 zurück in das Sieb 12 gelangen, dann aber auf die inzwischen geschlossen Rückschlagklappe 26 treffen, wodurch diese Bypass-Strömung 36 unterbrochen und somit verhindert wird, daß Schwammgummikugeln 20 aus dem Siebkörper 12 zurück in die Eintrittsleitung 13 und den Kühlwasseraustrittstutzen 6 zurückgefördert werden.

Es reicht dabei also völlig aus, die Absperrklappe 17 umzustellen und die Pumpe 14 abzuschalten - was sehr leicht automatisiert werden kann - ohne daß weitere Ventile betätigt werden müssen. Erst wenn beispielsweise die Schleuse 11 durch Abnehmen des oberen Deckels 37 geöffnet und die Schwammgummikugeln 20 entnommen werden sollen, ist es zweckmäßig, zusätzlich noch die Ventile 18 und 15 zu schließen, was von Hand oder mit Hilfe eines Stellmotors geschehen kann.

Im Normalfall werden dann die Schwammgummikugeln 20 dem Kühlwasserkreislauf dadurch wieder zugeführt, daß die Pumpe 14 eingeschaltet und die Absperrklappe 17 in die entsprechende Stellung zur Verbindung des Siebauslaßstutzens 16 und der Austrittsleitung 19 gefahren wird.

Mit der beschriebenen Anordnung und dem Einbau einer Rückschlagklappe in die Rückführleitung für die Reinigungskörper ist also auf einfache Weise eine äußerst funktionssichere und praktisch störungsfreie Lösung gegeben, die die bisher erforderlichen Manipulationsvorgänge herkömmlicher Reinigungskörper-Schleusen erheblich herabsetzt.

Taprogge Gesellschaft mbH       München, den 2.7.1983
4000 Düsseldorf 31              Mein Zeichen: PT 82/71 EP

Patentansprüche

1. Schleuse zum Auffangen von Reinigungskörpern in Form
von Schwammgummikugeln, die im Kreislauf vom Kühlwasser
durch die Röhren eines Wärmetauschers, insbesondere
Kraftwerkskondensators, geführt sind, wobei die Schleuse
aus einem in einem zylindrischen Gehäuse angeordneten
Siebkörper mit einer nachgeschalteten Absperrklappe besteht und über eine mit einer Pumpe versehenen Eintrittsleitung mit dem Austrittsstutzen des Wärmetauschers und
über eine mit einem Absperrventil versehene Austrittsleitung mit dem Eintrittsstutzen des Wärmetauschers in
Verbindung steht, d a d u r c h   g e k e n n z e i c h -
n e t , daß in der die Schleuse (10) durchsetzenden
Rückführleitung (13,19) für die Reinigungskörper (20)
zwischen Austrittsstutzen (6) und Eintrittsstutzen (1)
des Wärmetauschers (2) eine Rückschlagklappe (22;22';26)
angeordnet ist.

2. Schleuse nach Anspruch 1, dadurch gekennzeichnet,
daß die Rückschlagklappe (26) innerhalb des Schleusengehäuses (11) zur Absperrung der Eintrittsleitung (13)
angeordnet ist.

3. Schleuse nach Anspruch 2, dadurch gekennzeichnet,
daß die Rückschlagklappe (26) am innenliegenden Ende
eines in die Mündung (25) der Eintrittsleitung (13) eingeschobenen Rohrstutzens (31) schwenkbar befestigt ist.

4. Schleuse nach Anspruch 2, dadurch gekennzeichnet,
daß die Rückscklagklappe (26) im Bereich der Mündung (25)
der Eintrittsleitung (13) an der Gehäusewandung (11)
schwenkbar befestigt ist.

5.   Schleuse nach Anspruch 3, dadurch gekennzeichnet, daß der Rohrstutzen(31) an einem Ende einen rechteckigen Flansch (32) aufweist, an dessen oberer Kante die ebenfalls rechteckige Rückschlagklappe (26) in entsprechenden Scharnieren (33, 34) schwenkbar aufgehängt ist.

6.   Schleuse nach Anspruch 3 oder 4, dadurch gekennzeichnet, daß der Rohrstutzen (31) in Längsrichtung verlaufende Schlitze (35) aufweist und in der Mündung (25) der Eintrittsleitung (13) elastisch federnd verklemmt ist.

7.   Schleuse nach Anspruch 3, dadurch gekennzeichnet, daß der Rohrstutzen (31) mit der Eintrittsleitung (13) verschraubt ist.

8.   Schleuse nach Anspruch 3 oder 4, dadurch gekennzeichnet, daß die Schließbewegung der Rückschlagklappe (26) durch Federkraft unterstützt ist.

# FIG. 1

# FIG. 2

FIG.3

FIG.4

FIG.5A

FIG.5B

FIG.6